# EUROPEAN PATENT SPECIFICATION

(11) **EP 0 799 597 B1**
(45) Date of publication and mention of the grant of the patent: **22.11.2000**
(21) Application number: 96104356.9
(22) Date of filing: 19.03.1996
(51) Int. Cl.: A61B 5/00, A61N 1/36

(54) **Balance prothesis apparatus and method**
Verfahren und Vorrichtung für Gleichgewicht-Prothese
Procédé et appareil de prothèse d'équilibre

(43) Date of publication of application: 08.10.1997
(73) Proprietor: Balance International Inc., Boulder, Colorado 80302 (US)
(72) Inventor: Allum, John H.J., 4056 Basel (CH); Allum, Dianne J., 3807 Iseltwald (CH)
(74) Representative: Müller, Frithjof E., Dipl.-Ing.

(56) References cited:
- WO-A-88/04909
- DE-A- 3 416 873
- US-A- 4 092 633
- US-A- 5 281 957
- US-A- 5 361 778
- US-A- 5 361 788
- US-A- 5 469 861

## Description

This invention concerns a prosthetic apparatus which would augment the balance signals normally used by the brain to control the body in a stable position and prevent a fall and a balance prosthesis method of merely providing information to a subject on its state of balance.

Quantitative information on the efficacy of the human sense of balance can at present be obtained for medical purposes using eye movements or postural responses of the limbs measured electrophysiologically to determine if a response is outside of limits expected for individuals with a normal balance function. In the case when an individual has eye movements or postural responses outside normal limits then medication is given to that individual to reduce the action of peripheral senses on the brain or the individual takes part in a course of physical therapy over several weeks with the objective of training the brain to deal with a reduced sense of balance when trying to maintain the body upright and preventing a fall. However, these techniques do not fully meet the requirements of the clinician for a technique which would immediately augment the balance signals used by the brain. Difficulties arise with the presently available techniques mentioned above because the first kind, medication, can also reduce the capability of the brain to process balance information from the peripheral senses (vestibular, proprioceptive and visual) and because the second kind requires a long training period over more than two months. These difficulties are most evident when the subject is older than 65 years of age and likely to have a falling tendency, and when it would be advisable for a control monitoring station or a relative of the subject to know if the subject was in danger of falling.

In the field of hearing which is physiologically related to that of balance two types of prosthesis exist to augment the sense of hearing. The first kind involves augmenting the sound waves in the external ear canal so that these have greater excursions when they reach the inner ear to be transduced into electrical signals reaching the brain. The second kind involves direct electrical stimulation to the inner ear. For the sense of balance for which sensory signals from different neurophysiological systems, including a major input from the vestibular system of the inner ear, are combined by the brain to yield a unitary sense of balance no prosthetic device exists.

Prior art document US-A-4,092,633 discloses a condition sensor system comprising a condition detector which produces a pulse when a parameter of a monitored condition exceeds a desired threshold. A resettable condition counter counts each pulse. A resettable timer is preset to produce a particular time frame. The counter produces a condition signal when the accumulated number of pulses within the time frame is equal to or greater than a preset count. Control means responsive to the incoming pulses and to the condition signal produce control signals that control utilization devices. After a suitable delay, the last detected pulse simultaneously resets the pulse counter and the timer, and prepares them for sensing another condition occurrence within the time frame.

US-A-5 361 778 discloses an apparatus and method for evaluating foot borne motion in an individual.

Further, prior art document US-A-5,469,861 discloses a method and apparatus for monitoring posture wherein a first unit is attached to a first position on a user's body, and a second unit is attached to a second position on the user's body. A signal such as an ultrasonic signal is transmitted by the first unit to the second unit. In response, the second unit transmits a signal back to the first unit. The round-trip travel time of the signals is proportional to the distance between the units. If the distance differs from an ideal distance by more than a maximum allowable amount, improper posture is indicated to the user by an alarm signal.

Prior art document WO-A-8 804 909 describes an apparatus and method for determining the presence of vestibular pathology. This apparatus consists of a support surface on which a subject stands erect. The surface is rotatable around an axis co-linear with ankle joints and rests on vertical force transducers, the signals from which are monitored by a computer. The subject's field of view is substantially surrounded by an enclosure which is also rotatable about the axis. The computer controls a stimulator of an inner ear vestibular system and monitors any resulting change in posture of the subject, moving the support as necessary. The computer then calculates whether or not the stimulus produces correlated and significant increases in one or more of the measured variables.

Prior art document DE-A-3 416 837 discloses a system for achieving an erect walking posture which uses sensors, a computer and stimulators for nerves.

Finally, prior art document US-A-5,281,957 discloses a portable computer and head mounted display wherein the display is constructed in a manner similar to a pair of eyeglasses but with liquid crystal display screens replacing or forming a portion of the eyeglass lenses. The display screens can be either opaque or light transmitting, and can be hinge mounted to the glasses frame so that they can be swung up and out of the field of vision of the user. Specialized uses for the portable computer and head mounted display include a computer desk for secretarial workstations and the like, a research library workstation, and a moving map installation in an automobile or airplane.

It is an object of the present invention to provide a prosthetic apparatus which does not reduce the capability of the brain to process balance information from the peripheral senses (vestibular, proprioceptive and visual) and which requires no long training period over more than two months and therewith improves the current situation, and a balance prosthesis method of merely providing information to a subject on its state of balance.

This object is solved with an apparatus according to claim 1 and a method according to claim 10. Preferred embodiments of the apparatus and the method of the invention are defined in the respective dependent claims.

The invention has arisen from two findings, both contrary to present understanding. The first finding is that the unitary sense of balance computed by the human brain is directed towards monitoring the upper body with a minimum angular velocity within a cone of angular stability. Once this cone of stability is exceeded with an excessive angular velocity, normal individuals will respond by correcting the trunks position within 200 msecs. The second finding concerns the relationships between angular and linear accelerations of the head sensed by the vestibular system in the inner ear, and angular and linear accelerations of the trunk during balance disturbances. These two findings are utilized in the current invention to develop the method and apparatus for a balance prosthesis.

Accordingly, in a more general form thereof, a method and an apparatus for a balance prosthesis comprise a means for measuring the angular position and rate of the trunk, a means for comparing the current angular position and rate of the trunk to known safe limits, means for providing the information measured via any of four different sensory modalities and its relation to the safety limits to the subject, and means for providing the aforementioned measured information, safety limits and the functional status of the apparatus used to provide this information to the subject to any monitoring station interested in knowing the subject's state of balance.

The illustrated form of the apparatus according to the invention is of the more particular form mentioned above and comprises an angular rate and position transducer 1 worn by the subject on his/her upper body firmly attached to the chest or lower neck. This transducer measures the angular rate and position in pitch (fore-aft), roll (side-to-side) and, if necessary, in the yaw direction (turning about the vertical axis) as illustrated by the arrow 2. The technique used to measure the angular rate or position of the subject's trunk with respect to the earth's coordinates is not specific for the invention. The angular position, rate, or acceleration could be measured and then integrated or differentiated respectively to yield angular rate and position. In another form of the transducer linear acceleration transducers can be used as illustrated by the arrow 3. In this latter form- pairs of linear acceleration transducers can be used to generate angular acceleration measurements. In a further form the transducer 1 can be firmly attached to the head either by placing it in an ear mould in the external ear canal or it can be attached to a pair of spectacles. The angular rate and position signals from the head-mounted transducer would then be transformed to trunk rate and position in a microprocessor 4 or other equivalent electronics using algorithms that relate the angular rate and position of the head to those of the trunk during balance perturbations.

The processor 4 carries a detector and transformer stages. As indicated above, the detector stages examine angular velocity and position signals separately for rotations in pitch, yaw and roll planes and detect if either a rotation angle or velocity exceeds safety limits previously set in the processor 4. On the basis of the development of the invention so far it can be expected that the safety limits for backwards pitching will be 4 deg and 30 deg/sec and for forwards pitching 6 deg and 100 deg/sec. The detailed form of the safety limits in each of the rotation planes appear to very uniquely between individual patients requiring a test of the patient's balance capability to be built into the processor 4 rather than relying on a standard set of safety limits.

The transformer stage converts the angular rate and position signals from the transducer 1 as appropriate for the mode of stimulation (visual, vibrotactile, aural or electrical) used by the wearable stimulator 5. In the current form of the apparatus of the invention for vibro-tactile stimulation three vibrators are used to convey a sense of rotation in one direction. Two vibrators operate at 250 Hz, each conveying, for example, a sense of forwards and backwards sway with a modulation of the frequency of vibration corresponding to the velocity of sway and the amplitude of vibration corresponding to the angle of sway. The third vibrator In this form of the invention is used to signal the fact that sway has exceeded the limits of safety.

In another development of the invention the position and angular rate of the trunk is displayed to the subject in a head-mounted visual see-through display. In this form of the invention exceeding the safety limits is conveyed to the subject using repetitive changes in intensity of the display.

In a further development of the invention the position and angular rate of the trunk is transformed into a varying auditory signal the subject hears. In this form of the invention exceeding the safety limits or a sense of motion of the trunk or head is conveyed to the subject using the emission of sound.

In a still further development of the apparatus according to the invention the position and angular rate of the trunk is transformed into a varying electrical signal directly stimulating the vestibular nerve and sensed as a change in an angular and/or linear position of the head. In this form of the invention exceeding the safety limits is conveyed to the subject using direct electrical stimulation of the vestibular nerve of the subject in his/her inner ear, therefore control signals for such direct electrical stimulation are transmitted transcutaneously to an implantable device directly connected via electrodes to the close proximity of the vestibular nerve or to the nerve itself and the pulse rate and duty cycle of the electrical stimulation at the electrodes vary according to the amplitudes and frequencies produced while measuring the movements of the upper body.

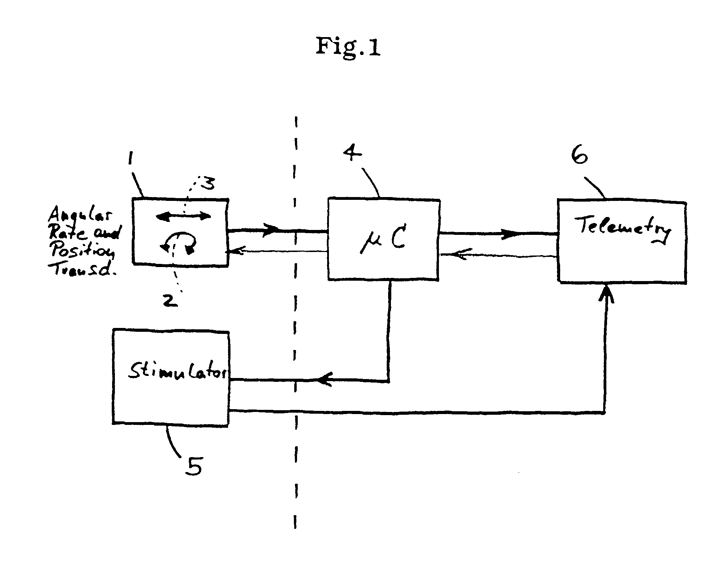

In a simple form of the apparatus, only the transducer and stimulator are worn by the subject as indicated by the dashed vertical line in Fig. 1. The signals from the transducer 1 and control signals to the stimulator 5 can be transmitted telemetrically to an external processor 4. Furthermore, the external processor can monitor telemetrically the status of the transducer 1 and/or stimulator 5. In a more sophisticated apparatus the processor 4 can also be worn by the subject. In an even more sophisticated device signals from the processor 4 and from the stimulator 5 can be passed telemetrically to and from a central monitoring station 6 which can control the state of instability of the wearer and whether the wearer is receiving adequate warning that he/she is in danger of falling.

Turning lastly to the question of practical application of the apparatus according to the invention: it is clear from the above discussion that the invention finds its primary application in augmenting the sense of balance of the elderly and others prone to fall, thereby helping to prevent a fall. Another application of the invention is to monitor the safety of a large number of patients who are in danger of falling as well as providing a record of how and when a patient in a home for the elderly actually fell. A third application is directly related to the individual limits of safety for impeding a fall of the subject. Modifications of these safety limits over time provides a way to test the efficacy of medication on balance function.

## Claims

1. Balance prosthesis apparatus comprising means (1) for measuring the angular velocity and position of the trunk of a subject in the fore-and-aft direction, side-to-side direction and the direction obtained by turning the trunk about a vertical axis of the subject, and means (5) for providing information on the changes in the amplitudes and frequency of these angular movements over time to the subject using either vibro-tactile, auditory, visual or direct electrical stimulation to the subject.

2. Apparatus according to claim 1, **characterized in** that said measuring means (1) includes motion measuring systems whose primary mode of operation is a measurement of changes in the angular position or velocity or acceleration of the trunk of the subject.

3. Apparatus according to claim 2, **characterized by** angular velocity transducers mounted on the trunk of the subject and capable of measuring angular velocities at least in the range of 0 to 100 deg/sec from 0 Hz up to a frequency of 100 Hz.

4. Apparatus according to anyone of claims 1 to 3, **characterized in** that the information gathered by said measuring means (1) is transformed to a varying vibro-tactile signal the subject feels by said means (5) for providing information to the subject consisting on one or more vibro-tactile devices placed on the skin of the subject either on the chest or on the back of the neck, or on the upper legs and capable of transmitting a sense of motion of the trunk to the subject using vibration.

5. Apparatus according to anyone of claims 1 to 3, **characterized in** that the information gathered by said measuring means (1) is transformed to a varying auditory signal the subject hears by the means for providing information to the subject consisting of an aural probe for insertion in the external canal of the subject's ear and said probe is capable of emitting sound conveying a sense of motion of the trunk to the subject.

6. Apparatus according to anyone of claims 1 to 3, **characterized in** that the information gathered by said measuring means (1) is transformed to a visual signal the subject sees by said means (5) for providing information to the subject consisting of a see-through head-mounted display in which the subject sees a visual display of the position or velocity of the trunk with either the left or right eye or both eyes and thereby visually senses motion of the trunk.

7. Apparatus according to anyone of claims 1 to 3, **characterized in** that the information gathered by said measuring means (1) is transformed to a varying electrical signal directly stimulating the vestibular nerve and sensed as a change in an angular and/or linear position of the head by the means (5) for providing information to the subject consisting of a means for direct electrical stimulation of the vestibular nerve of the subject in his/her inner ear, wherein control signals for such direct electrical
stimulation are transmitted transcutaneously to an implantable device directly connected via electrodes to the close proximity of the vestibular nerve or to the nerve itself, and the pulse rate and duty cycle of the electrical stimulation at the electrodes vary according to the amplitudes and frequencies measured by said measuring means.

8. Apparatus according to anyone of claims 1 to 7, **characterized by** a processor (4) in which a set of safety rules is determined for each axis of turning of the body consisting of limits of angular velocity and angular position separate for each axis in the positive and negative direction which the trunk must not exceed if a fall is to be avoided.

9. Apparatus according to claim 8, **characterized by** transmitting means which transmit the status of the trunk movements when exceeding said safety rules and the status of said means (5) for providing information to the subject or to a central monitoring station telemetrically.

10. Balance prosthesis method of merely providing information to a subject on its state of balance said method comprising steps of providing information on the changes in the amplitudes and frequency of angular trunk movements over time to the subject using either vibro-tactile, auditory or visual stimulation to the subject, thereby inducing a reduction in trunk movements to be within safe limits.

11. Method according to claim 10, characterized in that said movements of the trunk are primary measured as angular changes in the position or velocity or acceleration of the trunk of the subject.

12. Method according to claim 11, characterized by measuring angular velocities at least in the range of 0 to 1000 deg/sec from 0 Hz up to a frequency of 100 Hz.

13. Method according to anyone of claims 10 to 12, characterized in that the information about the angular movements of the trunk is transformed to a) a varying vibro-tactile signal the subject feels, or b) a varying auditory signal the subject hears, or c) a visual signal the subject sees, and sensed as a change in an angular and/or linear position of the head.

14. Method according to anyone of claims 10 to 13, characterized in that a set of safety rules is determined for each axis of turning of the body consisting of limits of angular velocity and angular position separate for each axis in the positive and negative direction which the trunk must not exceed if a fall is to be avoided.

15. Method according to claim 14, characterized in that the status of the trunk movements exceeding said safety rules and the status of the transformation of the information about the movements of the trunk are transmitted to a central monitoring station telemetrically.

## Patentansprüche

1. Gleichgewicht-Prothese-Vorrichtung, umfassend: eine Einrichtung (1) zum Messen der Winkelgeschwindigkeit und Position des Rumpfes einer Person in deren Vorwärts- und Rückwärts-Richtung, deren Seite-zu-Seite-Richtung und der durch Drehen des Rumpfes um eine vertikale Achse der Person erhaltenen Richtung und eine Einrichtung (5), die zeitabhängig Information über die Änderungen in den Amplituden und der Frequenz dieser Winkelbewegungen an die Person liefert mittels entweder vibro-taktiler, auditorischer, visueller oder direkter elektrischer Stimulation an der Person.

2. Vorrichtung nach Anspruch 1, **dadurch gekennzeichnet**, dass die Messeinrichtung (1) Bewegungsmesssysteme umfasst, deren Primärmodus des Betriebs eine Messung von Änderungen in der Winkelposition oder -geschwindigkeit oder -beschleunigung des Rumpfes der Person ist.

3. Vorrichtung nach Anspruch 2, **gekennzeichnet durch** Winkelgeschwindigkeitswandler, die an dem Rumpf der Person angebracht sind und in der Lage sind, Winkelgeschwindigkeiten wenigstens in dem Bereich von 0 bis 100°/s von 0 Hz bis zu einer Frequenz von 100 Hz zu messen.

4. Vorrichtung nach einem der Ansprüche 1 bis 3, **dadurch gekennzeichnet**, dass die durch die Messeinrichtung (1) gewonnene Information in ein sich änderndes vibro-taktiles Signal transformiert wird, das die Person durch die Liefereinrichtung (5) für Information an die Person fühlt, welche aus einer oder mehreren vibro-taktilen Einheiten besteht, die auf der Haut der Person entweder auf dem Thorax oder auf der Rückseite des Halses oder auf dem Oberbereich der Beine angebracht sind und ein Bewegungsgefühl des Rumpfes an die Person mittels Vibration zu übertragen vermögen.

5. Vorrichtung nach einem der Ansprüche 1 bis 3, **dadurch gekennzeichnet**, dass die durch die Messeinrichtung (1) gewonnene Information in ein sich änderndes auditorisches Signal transformiert wird, das die Person durch die Liefereinrichtung für Information an die Person hört, die aus einer Ohrsonde zur Einführung in den externen Kanal des Personenohres besteht, wobei die Sonde in der Lage ist, einen Ton zu emittieren, der ein Bewegungsgefühl des Rumpfes an die Person vermittelt.

6. Vorrichtung nach einem der Ansprüche 1 bis 3, **dadurch gekennzeichnet**, dass die durch die Messeinrichtung (1) gewonnene Information in ein visuelles Signal transformiert wird, das die Person durch die Liefereinrichtung (5) für Information an die Person sieht, welche aus einer am Kopf montierten Durchsicht-Anzeige besteht, in welcher die Person eine visuelle Anzeige der Position oder Geschwindigkeit des Rumpfes mit entweder dem linken oder dem rechten Auge oder beiden Augen sieht und dadurch visuell die Bewegung des Rumpfes erfasst.

7. Vorrichtung nach einem der Ansprüche 1 bis 3, **dadurch gekennzeichnet**, dass die durch die Messeinrichtung (1) gewonnene Information in ein sich änderndes elektrisches Signal transformiert ist, das direkt den vestibulären Nerv stimuliert und als eine Änderung in einer Winkel- und/oder Linearposition des Kopfes durch die Liefereinrichtung (5) für Information an die Person erfasst ist, die aus einer Einrichtung zur direkten elektrischen Stimulation des vestibulären Nervs der Person in seinem/ihrem Innenohr besteht, wobei Steuersignale für eine derartige direkte elektrische Stimulation transkutan zu einer implantierbaren Einheit übertragen sind, die direkt mittels Elektroden in enger Nähe zu dem vestibulären Nerv oder mit dem Nerv selbst verbunden sind, und wobei die Pulsrate sowie der Tastzyklus der elektrischen Stimulation an den Elektroden sich gemäß den Amplituden und Frequenzen verändern, die durch die Messeinrichtung gemessen sind.

8. Vorrichtung nach einem der Ansprüche 1 bis 7, **gekennzeichnet durch** einen Prozessor (4), in welchem ein Satz von Sicherheitsregeln für jede Achse der Drehung des Körpers bestimmt ist, welcher aus Grenzen der Winkelgeschwindigkeit und Winkelposition getrennt für jede Achse in der positiven und negativen Richtung besteht, die der Rumpf nicht überschreiten darf, wenn ein Fallen zu vermeiden ist.

9. Vorrichtung nach Anspruch 8, **gekennzeichnet durch** eine Übertragungseinrichtung, die den Status der Rumpfbewegungen bei Überschreiten der Sicherheitsregeln und den Status der Liefereinrichtung (5) von Information an die Person oder an eine zentrale Überwachungsstation telemetrisch überträgt.

10. Gleichgewicht-Prothese-Verfahren, um lediglich Information an eine Person über deren Gleichgewichtszustand zu liefern, wobei das Verfahren Schritte des Lieferns von Information über die Änderungen in den Amplituden und der Frequenz von Winkel-Rumpf-Bewegungen über der Zeit an die Person mittels entweder vibro-taktiler, auditorischer oder visueller Stimulation zu der Person umfasst, um dadurch eine Reduktion in Rumpfbewegungen innerhalb sicherer Grenzen hervorzurufen.

11. Verfahren nach Anspruch 10, **dadurch gekennzeichnet**, dass die Bewegungen des Rumpfes primär als Winkeländerungen in der Position oder Geschwindigkeit oder Beschleunigung des Rumpfes der Person gemessen sind.

12. Verfahren nach Anspruch 11, **gekennzeichnet durch** ein Messen von Winkelgeschwindigkeiten wenigsten in dem Bereich von 0 bis 1000°/s von 0 Hz bis zu einer Frequenz von 100 Hz.

13. Verfahren nach einem der Ansprüche 10 bis 12, **dadurch gekennzeichnet**, dass die Information über die Winkelbewegungen des Rumpfes transformiert sind in (a) ein sich änderndes vibro-taktiles Signal, das die Person fühlt, oder (b) ein sich änderndes auditorisches Signal, das die Person hört, oder (c) ein visuelles Signal, das die Person sieht, und erfasst sind als eine Änderung in einer Winkel- und/oder Linearposition des Kopfes.

14. Verfahren nach einem der Ansprüche 10 bis 13, **dadurch gekennzeichnet**, dass ein Satz von Sicherheitsregeln bestimmt ist für jede Achse der Drehung des Körpers, der aus Grenzen einer Winkelgeschwindigkeit und Winkelposition besteht, getrennt für jede Achse in positiver und negativer Richtung, welche der Rumpf nicht überschreiten darf, wenn ein Fallen zu vermeiden ist.

15. Verfahren nach Anspruch 14, **dadurch gekennzeichnet**, dass der Status der Rumpfbewegungen, die die Sicherheitsregeln überschreiten, und der Status der Transformation der Information über die Bewegungen des Rumpfes telemetrisch zu einer zentralen Überwachungsstation übertragen sind.

## Revendications

1. Appareil de prothèse d'équilibre comprenant des moyens (1) pour mesurer la vitesse angulaire et la position du tronc d'un sujet dans la direction avant-arrière, la direction d'un côté à l'autre et la direction obtenue par rotation du tronc autour d'un axe vertical du sujet, et des moyens (5) pour fournir au sujet des informations sur les variations des amplitudes et de la fréquence de ces mouvements angulaires dans le temps, par stimulation vibro-tactile, auditive, visuelle ou électrique directe.

2. Appareil selon la revendication 1, caractérisé en ce que les dits moyens de mesure (1) comprennent des systèmes de mesure de mouvement dont le mode principal de fonctionnement est une mesure des variations de la position angulaire ou de la vitesse ou de l'accélération du tronc du sujet.

3. Appareil selon la revendication 2, caractérisé en ce que des transducteurs de vitesse angulaire sont montés sur le tronc du sujet et peuvent mesurer les vitesses angulaires au moins dans la plage de 0 à 100 degrés/seconde à partir de 0 Hz jusqu'à une fréquence de 100 Hz.

4. Appareil selon une quelconque des revendications 1 à 3, caractérisé en ce que l'information recueillie par les dits moyens de mesure (1) est transformée en un signal vibro-tactile variable que le sujet ressent, par les dits moyens (5) de fourniture d'information au sujet qui consistent en un ou plusieurs dispositifs vibro-tactiles placés sur la peau du sujet, soit sur la poitrine, soit sur la nuque, soit sur les cuisses, et pouvant transmettre une sensation de mouvement du tronc au sujet par utilisation d'une vibration.

5. Appareil selon une quelconque des revendications 1 à 3, caractérisé en ce que l'information recueillie par les dits moyens de mesure (1) est transformée en un signal auditif variable que le sujet entend, par les moyens de fourniture d'information au sujet qui consistent en une sonde d'oreille pour insertion dans le canal externe de roreille du sujet, la dite sonde pouvant émettre un son qui donne une sensation de mouvement du tronc au sujet.

6. Appareil selon une quelconque des revendications 1 à 3, caractérisé en ce que l'information recueillie par les dits moyens de mesure (1) est transformée en un signal visuel que le sujet voit, par les dits moyens (5) de fourniture d'information au sujet qui consistent en un affichage transparent fixé à la tête dans lequel le sujet voit une indication visuelle de la position ou de la vitesse du tronc avec l'oeil gauche ou l'oeil droit ou les deux yeux et ressent ainsi visuellement un mouvement du tronc.

7. Appareil selon une quelconque des revendications 1 à 3, caractérisé en ce que l'information recueillie par les dits moyens de mesure (1) est transformée en un signal électrique variable qui stimule directement le nerf vestibulaire et qui est ressenti comme un changement d'une position angulaire et/ou linéaire de la tête, par les moyens (5) de fourniture d'information au sujet qui consistent en un dispositif de stimulation électrique directe du nerf vestibulaire du sujet dans son oreille interne, dans lequel les signaux de commande de cette stimulation électrique directe sont transmis de façon transcutanée à un dispositif implantable directement connecté via des électrodes au voisinage immédiat du nerf vestibulaire ou au nerf lui-même, et la cadence et le cycle d'activité des impulsions de la stimulation électrique appliquée aux électrodes varient en fonction des amplitudes et des fréquences mesurées par les dits moyens de mesure.

8. Appareil selon une quelconque des revendications 1 à 7, caractérisé en ce qu'il comprend un processeur (4) dans lequel un ensemble de règles de sécurité est déterminé pour chaque axe de rotation du corps, ces règles consistant en limites de vitesse angulaire et de position angulaire séparées pour chaque axe dans la direction positive et négative qui ne doivent pas être dépassées par le tronc si on veut éviter une chute.

9. Appareil selon la revendication 8, caractérisé en ce qu'il comprend des moyens de transmission qui transmettent l'état des mouvements du tronc lorsqu'ils dépassent les dites règles de sécurité et l'état des dits moyens (5) de fourniture d'information au sujet ou à une station centrale de surveillance, par télémétrie.

10. Procédé de prothèse d'équilibre pour fournir de façon simple des informations à un sujet sur son état d'équilibre, le dit procédé comprenant des étapes de fourniture d'information sur les variations des amplitudes et de la fréquence des mouvements angulaires du tronc dans le temps au sujet par utilisation d'une stimulation vibro-tactile, auditive ou visuelle du sujet, de façon à induire une réduction des mouvements du tronc afin qu'ils soient à l'intérieur de limites de sécurité.

11. Procédé selon la revendication 10, caractérisé en ce que les dits mouvements du tronc sont principalement mesurés comme des changements angulaires de la position ou de la vitesse ou de l'accélération du tronc du sujet.

12. Procédé selon la revendication 11, caractérisé en ce qu'on mesure les vitesses angulaires au moins dans la plage de 0 à 100 degrés/seconde à partir de 0 Hz jusqu'à une fréquence de 100 Hz.

13. Procédé selon une quelconque des revendications 10 à 12, caractérisé en ce que l'information concernant les mouvements angulaires du tronc est transformée en (a) un signal vibro-tactile variable que le sujet ressent, ou (b) un signal auditif variable que le sujet entend, ou (c) un signal visuel que le sujet voit, et elle est ressentie comme un changement d'une position angulaire et/ou linéaire de la tête.

14. Procédé selon une quelconque des revendications 10 à 13, caractérisé en ce qu'un ensemble de règles de sécurité est déterminé pour chaque axe de rotation du corps et consiste en limites de vitesse angulaire et de position angulaire séparées pour chaque axe dans la direction positive et négative, qui ne doivent pas être dépassées si on veut éviter une chute.

15. Procédé selon la revendication 1, caractérisé en ce que l'état des mouvements du tronc dépassant les dites règles de sécurité et l'état de la transformation de l'information concernant les mouvements du tronc sont transmis à une station centrale de surveillance, par télémétrie.
